(19) Europäisches Patentamt
European Patent Office
Office européen des brevets

(11) Numéro de publication : **0 306 410 B1**

(12) **FASCICULE DE BREVET EUROPEEN**

(45) Date de publication du fascicule du brevet : **21.08.91 Bulletin 91/34**

(51) Int. Cl.$^5$ : **C07C 255/15,** C07C 69/73, C07C 219/08

(21) Numéro de dépôt : **88402201.3**

(22) Date de dépôt : **01.09.88**

(54) **Nouveaux monomères éthyléniques polyfonctionnels.**

Le dossier contient des informations techniques présentées postérieurement au dépôt de la demande et ne figurant pas dans le présent fascicule.

(30) Priorité : **02.09.87 FR 8712203**

(43) Date de publication de la demande : **08.03.89 Bulletin 89/10**

(45) Mention de la délivrance du brevet : **21.08.91 Bulletin 91/34**

(84) Etats contractants désignés : **AT BE CH DE ES FR GB GR IT LI LU NL SE**

(56) Documents cités : **EP-A- 0 185 234**

(56) Documents cités : **SYNTHESIS, no. 2, février 1981, pages 129-130, Georg Thieme Verlag, Stuttgart, DE; H. STETTER et al.: "Additionen von Aldehyden an aktivierte Doppelbindungen; XXVI. Herstellung und Reaktionen von 1,1-Diethoxy-2,5-dioxoalkanen"**

(73) Titulaire : **SOCIETE FRANCAISE HOECHST Société anonyme dite: 3, avenue du Général de Gaulle F-92800 Puteaux (FR)**

(72) Inventeur : **Wilhelm, Didier 26 rue Diderot F-92130 Issy Les Moulineaux (FR)**
Inventeur : **Blanc, Alain 21 rue Galvanis F-75017 Paris (FR)**

(74) Mandataire : **Rinuy, Santarelli 14, avenue de la Grande Armée F-75017 Paris (FR)**

**EP 0 306 410 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux monomères éthyléniques polyfonctionnels.

Le document EP-A-185234 révèle une série de composés répondant à la formule générale (IV) :

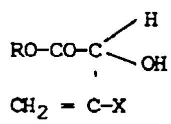

(IV)

où R = alkyl en $C_1$-$C_{18}$

X = —CN, 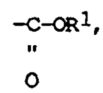

$R^1$ = alkyl en $C_1$-$C_{18}$, $-(CH_2)_n-OH$, 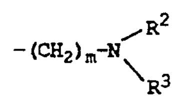 ,

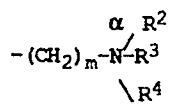 $Y^{\ominus}$, $-(C_2H_4O)_pR$

$R^2$, $R^3$ = —$CH_3$, —$C_2H_5$

$R^4$ = $-H$, $-CH_3$, $-C_2H_5$, 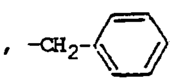

n = 2 à 4,
m = 2 à 5,
p = 1 à 80, et
$Y^-$ = $Cl^-$, $Br^-$, $SO_4^{2-}$ ; $PO_4^{3-}$ ; $CH_3OSO_3^-$, $C_2H_5OSO_3^-$, $CH_3COO^-$, $HCOO^-$.

Ces nouveaux monomères éthyléniques polyfonctionnels répondant à la formule générale (I) :

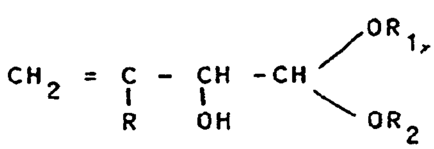

(I)

dans laquelle :

— R représente un groupement cyano, acyle substitué ou non, alcoxycarbonyle substitué ou non, ou aryloxycarbonyle substitué ou non,

— $R_1$ et $R_2$ sont identiques et ils représentent alors chacun un groupement —$CHR_3R_4$ dans lequel $R_3$ et $R_4$, identiques ou différents, représentent un atome d'hydrogène ou un radical alkyle, alcényle ou aralkyle, ou bien

— $R_1$ et $R_2$ forment ensemble un radical bivalent —$CH_2$-$(CR_5R_6)_n$-$CHR_7$ dans lequel n représente 0 ou 1 et $R_5$, $R_6$ et $R_7$, identiques ou différents, représentent un atome d'hydrogène ou un groupement méthyle.

Le terme acyle substitué ou non peut désigner par exemple un radical acétyle, propionyle, benzoyle, phénylacétyle.

Le terme alcoxycarbonyle substitué ou non peut désigner par exemple un radical méthoxycarbonyle, éthoxycarbonyle, propoxycarbonyle, butoxycarbonyle, diméthylaminoéthoxycarbonyle, hydroxyéthoxycarbonyle.

Le terme aryloxycarbonyle substitué ou non peut désigner par exemple un radical phénoxycarbonyle.

Le terme alkyle peut désigner par exemple un radical méthyle, éthyle, n-propyle, isopropyle, n-butyle, isobutyle, méthyl-1 propyle, éthyl-1 propyle.

Le terme alcényle peut désigner par exemple un radical allyle, méthallyle.

Le terme aralkyle peut désigner par exemple un radical benzyle éventuellement substitué sur le noyau aromatique, un radical phénéthyle éventuellement substitué sur le noyau aromatique.

L'invention a plus particulièrement pour objet les produits tels que définis ci-dessus, caractérisés en ce que dans la formule (I), R représente un groupement cyano ou un groupement alcoxycarbonyle substitué ou non et $R_1$ et $R_2$, lorsqu'ils sont identiques, représentent chacun un groupement méthyle, éthyle ou butyle, ou bien, $R_1$ et $R_2$ forment ensemble un radical bivalent tel qu'éthylène, triméthylène, propylène ou diméthyl-2,2 propanediyl-1,3.

Parmi ces derniers produits, l'invention couvre notamment :
- le cyano-2 diméthoxy-4,4 hydroxy-3 butène-1,
- le cyano-2 dibutoxy-4,4 hydroxy-3 butène-1,
- le dibutoxy-4,4 éthoxycarbonyl-2 hydroxy-3 butène-1,
- le butoxycarbonyl-2 dibutoxy-4,4 hydroxy-3 butène-1,
- le diméthoxy-4,4 hydroxy-3, hydroxyéthoxycarbonyl-2 butène-1.
- le diméthoxy-4,4 diméthylaminoéthoxycarbonyl-2 hydroxy-3 butène-1,
- le diméthoxy-4,4 éthoxycarbonyl-2 hydroxy-3 butène-1.

Les produits de formule (I) ci-dessus peuvent être préparés par des procédés connus en eux-mêmes consistant à faire réagir le monoacétal du glyoxal de formule (II) :

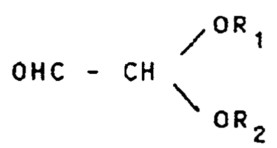

(II)

(dans laquelle $R_1$ et $R_2$ ont la signification donnée précédemment), avec un dérivé éthylénique de formule (III)

$$H_2C = CH - R \quad (III)$$

(dans laquelle R a la signification donné précédemment), en présence d'un catalyseur alcalin aprotique choisi avantageusement parmi les amines tertiaires. Préférentiellement, ce catalyseur alcalin est le diazabicyclo [2,2,2] octane-1,4, désigné ci-après DABCO.

De tels procédés sont notamment décrits dans les brevets suivants : US 3.499.024 ; US.3.743.669 ; US 4.652.669 ; FR 1.506.132 ; FR 2.120.686 ; EP 185.234 ; EP 196.708.

Les produits de formule générale (I) sont des monomères polyfonctionnels précieux susceptibles d'être utilisés par exemple dans la préparation de polymères.

Les exemples suivants sont donnés à titre illustratif et nullement limitatif :

Exemple 1

On abandonne 9 jours sous agitation à la température ambiante une solution constituée par :
- 15,8 (0,30 mole) d'acrylonitrile,
- 25 g (0,22 mole) de diméthoxyéthanal à 90% en poids dans du méthanol,
- 2,5 g (22 mmoles) de DABCO,
- 6 mg de paraméthoxyphénol.

Cette solution diluée avec 150 g de diéthyle oxyde est ensuite lavée avec 100 g d'acide chlorhydrique 1N puis la phase aqueuse est lavée deux fois avec 150 g de diéthyle oxyde. Les phases organiques réunies sont ensuite lavées à l'eau jusqu'à neutralité des lavages puis elles sont séchées sur sulfate de magnésium anhydre, filtrées et concentrées sous vide. L'huile incolore résiduelle est enfin distillée sous vide.

On obtient ainsi 17,58 g de cyano-2 diméthoxy-4,4 hydroxy-3 butène-1 distillant sous 0,05 mbar à 80 ± 3°C.

Microanalyse

| | | C % | H % | N % | O % |
|---|---|---|---|---|---|
| $C_7H_{11}NO_3$ | calculés | 53,5 | 7,05 | 8,9 | 30,55 |
| PM = 157,17 | trouvés | 53,8 | 7,1 | 8,7 | |

RMN[1]H dans le $CDCl_3$ à 200 MHz

6,12 ppm (m, 2H, $CH_2=$)
4,33 ppm (d, 1H, J = 5,8 Hz $\underline{CH}(OMe)_2$)
4,19 ppm (m, 1H, —$\underline{CH}OH$)
3,48 ppm (s, 3H, $OCH_3$)
3,47 ppm (s, 3H, $OCH_3$)
2,86 ppm (m, 1H, OH)

RMN[13]C dans le $CDCl_3$ à 50 Mhz

132,8 ppm $\underline{C}H_2=$
122 ppm $=\underline{C}$
116,8 ppm $\underline{C}\equiv N$
104,9 ppm $\underline{C}H(OMe)_2$
71,8 ppm $\underline{C}HOH$
56 ppm $O\underline{C}H_3$
55,2 ppm $O\underline{C}H_3$

## Exemple 2

En opérant comme décrit à l'exemple 1 au départ de 0,17 mole d'acrylonitrile et de 30 g (0,15 mole) de dibutoxyéthanal à 95,5% en poids dans du butanol, on obtient 22 g de cyano-2 dibutoxy-4,4 hydroxy-3 butène-1 distillant à 108°C sous 0,1 mbar.

## Exemple 3

En opérant comme décrit à l'exemple 1 au départ de 118 mmoles d'acrylate d'éthyle et de 10 g (86,5 mmoles) de diméthoxyéthanal à 90% en poids dans du méthanol, on obtient le diméthoxy-4,4 éthoxycarbonyl-2 hydroxy-3 butène-1 sous forme d'un liquide distillant à 108°C sous 0,3 mbar.

Microanalyse

| | | C % | H % | O % |
|---|---|---|---|---|
| $C_9H_{16}O_5$ | calculés | 52,94 | 7,89 | 39,17 |
| PM = 204,2 | trouvés | 52,6 | 7,9 | |

## Exemple 4

En opérant comme décrit à l'exemple 1 au départ de 0,4 mole d'acrylate de butyle et de 70 g (0,355 mole) de dibutoxyéthanal à 95,5% en poids dans du butanol, on obtient le butoxycarbonyl-2 dibutoxy-4,4 hydroxy-3 butène-1 sous forme d'un liquide incolore distillant à 130°C sous 0,2 mbar.

Microanalyse

| | | C % | H % | O % |
|---|---|---|---|---|
| $C_{17}H_{32}O_5$ | calculés | 64,5 | 10,2 | 25,3 |
| PM = 316,44 | trouvés | 64,6 | 10,2 | |

Exemple 5

On chauffe 5 heures à 70°C sous agitation une solution constituée par :
— 4,06 g (35 mmoles) d'acrylate d'hydroxy-2 éthyle,
— 3 g (26 mmoles) de diméthoxyéthanal à 90% en poids dans du méthanol,
— 400 mg de DABCO,
— 2 mg de paraméthoxyphénol.

Puis la solution est chromatographiée sur de la silice avec élution avec un mélange dichlorométhaneméthanol, 9/1 v/v et les éluats sont ensuite distillés sous vide. On obtient ainsi le diméthoxy-4,4 hydroxy-3 hydroxyéthoxycarbonyl-2 butène-1 distillant à 185 ± 5°C sous 5 mbar.

Microanalyse

| | | C % | H % | O % |
|---|---|---|---|---|
| $C_9H_{16}O_6$ | calculés | 49,1 | 7,3 | 43,6 |
| PM = 220,22 | trouvés | 49,1 | 7,4 | |

Exemple 6

On chauffe 120 heures à 50°C sous agitation une solution constituée par :
— 5,01 g (35 mmoles) d'acrylate de diméthylaminoéthyle,
— 3 g (26 mmoles) de diméthoxyéthanal en solution dans du méthanol à 90% en poids,
— 400 mg de DABCO,
— 2 mg de paraméthoxyphénol.

Puis la solution obtenue est filtrée sur silice avec élution avec un mélange dichlorométhane — méthanol 9/1 v/v. Les éluats sont ensuite réunis puis concentrés sous vide. L'huile est alors distillée sous vide. On obtient ainsi 4,5 de diméthoxy-4,4 diméthylaminoéthoxycarbonyl-2 hydroxy-3 butène-1 distillant à 160 ± 5°C sous 5 mbar.

Microanalyse

| | | C % | H % | N % | O % |
|---|---|---|---|---|---|
| $C_{11}H_{21}NO_5$ | calculés | 53,4 | 8,6 | 5,7 | 32,3 |
| PM = 247,29 | trouvés | 53,4 | 8,6 | 5,6 | |

Exemple 7

En opérant comme décrit à l'exemple 1 au départ de 400 mmoles d'acrylate d'éthyle et de 70 g (355 mmoles) de dibutoxyéthanal à 95,5% en poids dans du butanol, on obtient le dibutoxy-4,4 éthoxycarbonyl-2 hydroxy-3 butène-1 sous forme d'un liquide distillant à 120°C sous 0,4 mbar.

## Revendications

### Revendications pour les Etats contractants suivants : AT, BE, CH, DE, FR, GB, GR, IT, LI, LU, NL, SE

1. Un monomère éthylénique polyfonctionnel de formule générale I :

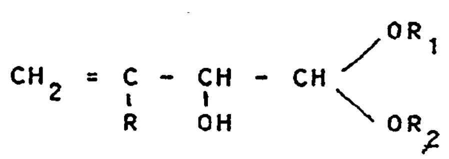

(I)

dans laquelle :

— R représente un groupement cyano, acyle substitué ou non, alcoxycarbonyle substitué ou non ou aryloxycarbonyle substitué ou non,

— $R_1$ et $R_2$ sont identiques et ils représentent chacun un groupement —$CHR_3R_4$ dans lequel $R_3$ et $R_4$, identiques ou différents, représentent un atome d'hydrogène ou un radical alkyle, alcényle ou aralkyle,

ou bien

— $R_1$ et $R_2$ forment ensemble un radical bivalent —$CH_2(CR_5R_6)_n$-$CHR_7$— dans lequel n représente 0 ou 1 et $R_5$, $R_6$ et $R_7$, identiques ou différents, représentent un atome d'hydrogène ou un groupement méthyle.

2. Un monomère éthylénique tel que défini à la revendication 1, caractérisé en ce que dans la formule (I), R représente un groupement cyano ou un groupement alcoxycarbonyle et $R_1$ et $R_2$, lorsqu'ils sont identiques, représentent chacun un groupement méthyle, éthyle ou butyle, ou bien $R_1$ et $R_2$ forment ensemble un radical bivalent tel qu'éthylène, triméthylène, propylène ou diméthyl-2,2 propanediyl-1,3.

3. Le cyano-2 diméthoxy-4,4 hydroxy-3 butène-1.

4. Le cyano-2 dibutoxy-4,4 hydroxy-3 butène-1.

5. Le dibutoxy-4,4 éthoxycarbonyl-2 hydroxy-3 butène-1.

6. Le butoxycarbonyl-2 dibutoxy-4,4 hydroxy-3 butène-1.

7. Le diméthoxy-4,4 hydroxy-3 hydroxyéthoxycarbonyl-2 butène-1.

8. Le diméthoxy-4,4 diméthylaminoéthoxycarbonyl-2 hydroxy-3 butène-1.

9. Le diméthoxy-4,4 éthoxycarbonyl-2 hydroxy-3 butène-1.

**Revendications pour l'Etat contractant suivant : ES**

1. Procédé de préparation de monomères éthyléniques polyfonctionnels de formule générale I :

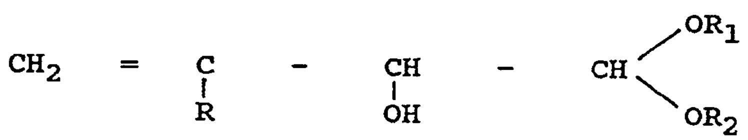

(I)

dans laquelle :

— R représente un groupement cyano, acyle substitué ou non, alcoxycarbonyle substitué ou non ou aryloxycarbonyle substitué ou non,

— $R_1$ et $R_2$ sont identiques et ils représentent chacun un groupement —$CHR_3R_4$ dans lequel $R_3$ et $R_4$, identiques ou différents, représentent un atome d'hydrogène ou un radical alkyle, alcényle ou aralkyle,

ou bien

— $R_1$ et $R_2$ forment ensemble un radical bivalent —$CH_2(CR_5R_6)_n$ -$CHR_7$— dans lequel n représente 0 ou 1 et $R_5$, $R_6$ et $R_7$, identiques ou différents, représentent un atome d'hydrogène ou un groupement méthyle,

caractérisé en ce que l'on fait réagir le monoacétal du glyoxal de formule (II) :

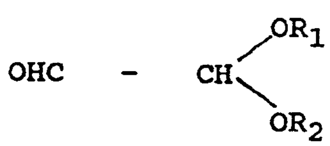

(II)

dans laquelle $R_1$ et $R_2$ ont la signification donnée précédemment avec un dérivé éthylénique de formule (III) :

$$H_2C = CH - R \qquad (III)$$

dans laquelle R a la signification donnée précédemment, en présence d'un catalyseur alcalin aprotique.

2. Procédé selon la revendication 1, caractérisé en ce que le catalyseur est le diazabicyclo[2,2,2]octane-1,4.

3. Procédé selon la revendication 1 ou 2, caractérisé en ce que l'on prépare un dérivé de formule I dans laquelle R représente un groupement cyano ou un groupement alcoxycarbonyle et $R_1$ et $R_2$, lorsqu'ils sont identiques, représentent chacun un groupement méthyle, éthyle ou butyle, ou bien $R_1$ et $R_2$ forment ensemble un radical bivalent tel qu'éthylène, triméthylène, propylène ou diméthyl-2,2 propanediyl-1,3.

4. Procédé selon la revendication 1 ou 2, caractérisé en ce que l'on prépare le cyano-2 diméthoxy-4,4 hydroxy-3 butène-1.

5. Procédé selon la revendication 1 ou 2, caractérisé en ce que l'on prépare le cyano-2 dibutoxy-4,4 hydroxy-3 butène-1.

6. Procédé selon la revendication 1 ou 2, caractérisé en ce que l'on prépare le dibutoxy-4,4 éthoxycarbonyl-2 hydroxy-3 butène-1.

7. Procédé selon la revendication 1 ou 2, caractérisé en ce que l'on prépare le butoxycarbonyl-2 dibutoxy-4,4 hydroxy-3 butène-1.

8. Procédé selon la revendication 1 ou 2, caractérisé en ce que l'on prépare le diméthoxy-4,4 hydroxy-3 hydroxyéthoxycarbonyl-2 butène-1.

9. Procédé selon la revendication 1 ou 2, caractérisé en ce que l'on prépare le diméthoxy-4,4 diméthylaminoéthoxycarbonyl-2 hydroxy-3 butène-1.

10. Procédé selon la revendication 1 ou 2, caractérisé en ce que l'on prépare le diméthoxy-4,4 éthoxycarbonyl-2 hydroxy-3 butène-1.

**Patentansprüche**

**Patentansprüche für folgende Vertragsstaaten : AT, BE, CH, DE, FR, GB, GR, IT, LI, LU, NL, SE**

1. Polyfunktionnelles Äthylenmonomer der allgemeinen Formel (I)

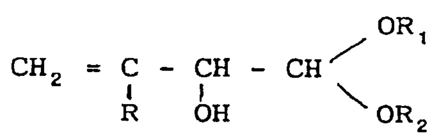

(I),

worin R eine Cyan-, gegebenenfalls substituierte Acyl-, gegebenenfalls substituierte Alkoxycarbonyl- oder gegebenenfalls substituierte Aryloxycarbonylgruppe bedeutet,

$R_1$ und $R_2$ gleich sind und jeweils eine Gruppe $-CHR_3R_4$ darstellen, worin $R_3$ und $R_4$, die gleich oder verschieden sind, ein Wasserstoffatom oder einen Alkyl-, Alkenyl- oder Aralkylrest bedeuten, oder

$R_1$ und $R_2$ miteinander einen zweiwertigen Rest $-CH_2(CR_5R_6)_n-CHR_7-$ bilden, worin n Null oder 1 ist und $R_5$, $R_6$ und $R_7$, die gleich oder verschieden sind, ein Wasserstoffatom oder eine Methylgruppe darstellen.

2. Äthylenmonomer nach Anspruch 1, dadurch gekennzeichnet, daß in Formel (I) R eine Cyan- oder Alkoxycarbonylgruppe bedeutet und $R_1$ und $R_2$, wenn sie gleich sind, eine Methyl-, Äthyl- oder Butylgruppe darstellen oder $R_1$ und $R_2$ miteinander einen zweiwertigen Rest, wie Äthylen, Trimethylen, Propylen oder 2,2-Dimethyl-1,3-propandiyl, bilden.

3. 2-Cyano-4,4-dimethoxy-3-hydroxy-1-buten.

4. 2-Cyano-4,4-dibutoxy-3-hydroxy-1-buten.

5. 4,4-Dibutoxy-2-äthoxycarbonyl-3-hydroxy-1-buten.

6. 2-Butoxycarbonyl-4,4-dibutoxy-3-hydroxy-1-buten.

7. 4,4-Dimethoxy-3-hydroxy-2-hydroxyäthoxycarbonyl-1-buten.

8. 4,4-Dimethoxy-2-dimethylaminoäthoxycarbonyl-3-hydroxy-1-buten.

9. 4,4-Dimethoxy-2-äthoxycarbonyl-3-hydroxy-1-buten.

**Patentansprüche fur folgenden Vertragsstaat : ES**

1. Verfahren zur Herstellung von polyfunktionellen Äthylenmonomeren der allgemeinen Formel (I)

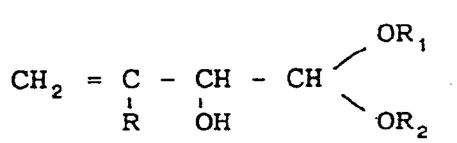

(I),

worin R eine Cyan-, gegebenenfalls substituierte Acyl-, gegebenenfalls substituierte Alkoxycarbonyl- oder

gegebenenfalls substituierte Aryloxycarbonylgruppe bedeutet,

$R_1$ und $R_2$ gleich sind und jeweils eine Gruppe —$CHR_3R_4$ darstellen, worin $R_3$ und $R_4$, die gleich oder verschieden sind, ein Wasserstoffatom oder einen Alkyl-, Alkenyl- oder Aralkylrest bedeuten, oder

$R_1$ und $R_2$ miteinander einen zweiwertigen Rest —$CH_2(CR_5R_6)_n$-$CHR_7$— bilden, worin n Null oder 1 ist und $R_5$, $R_6$ und $R_7$, die gleich oder verschieden sind, ein Wasserstoffatom oder eine Methylgruppe darstellen, dadurch gekennzeichnet, daß man das Monoacetal von Glyoxal der Formel (II)

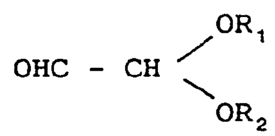

(II),

worin $R_1$ und $R_2$ die oben angegebene Bedeutung haben, mit einem Äthylenderivat der Formel (III)

$$H_2C = CH - R \qquad (III),$$

worin R die oben angegebene Bedeutung hat, in Anwesenheit eines aprotischen alkalischen Katalysators reagieren läßt.

2. Verfahren nach Anspruch 1, dadurch gekennzeichnet, daß der Katalysator 1,4-Diazabicyclo[2,2,2]octan ist.

3. Verfahren nach Anspruch 1 oder 2, dadurch gekennzeichnet, daß ein Derivat der Formel (I), worin R eine Cyan- oder Alkoxycarbonylgruppe bedeutet und $R_1$ und $R_2$, wenn sie gleich sind, eine Methyl-, Äthyl- oder Butylgruppe darstellen oder $R_1$ und $R_2$ miteinander einen zweiwertigen Rest, wie Äthylen, Trimethylen, Propylen oder 2,2-Dimethyl-1,3-propandiyl bilden, hergestellt wird.

4. Verfahren nach Anspruch 1 oder 2, dadurch gekennzeichnet, daß 2-Cyano-4,4-dimethoxy-3-hydroxy-1-buten hergestellt wird.

5. Verfahren nach Anspruch 1 oder 2, dadurch gekennzeichnet, daß 2-Cyano-4,4-dibutoxy-3-hydroxy-1-buten hergestellt wird.

6. Verfahren nach Anspruch 1 oder 2, dadurch gekennzeichnet, daß 4,4-Dibutoxy-2-äthoxycarbonyl-3-hydroxy-1-buten hergestellt wird.

7. Verfahren nach Anspruch 1 oder 2, dadurch gekennzeichnet, daß 2-Butoxycarbonyl-4,4-dibutoxy-3-hydroxy-1-buten hergestellt wird.

8. Verfahren nach Anspruch 1 oder 2, dadurch gekennzeichnet, daß 4,4-Dimethoxy-3-hydroxy-2-hydroxyäthoxycarbonyl-1-buten hergestellt wird.

9. Verfahren nach Anspruch 1 oder 2, dadurch gekennzeichnet, daß 4,4-Dimethoxy-2-dimethylaminoäthoxycarbonyl-3-hydroxy-1-buten hergestellt wird.

10. Verfahren nach Anspruch 1 oder 2, dadurch gekennzeichnet, daß 4,4-Dimethoxy-2-äthoxycarbonyl-3-hydroxy-1-buten hergestellt wird.

## Claims

### Claims for the following Contracting States : AT, BE, CH, DE, FR, GB, GR, IT, LI, LU, NL, SE

1. A polyfunctional ethylenic monomer having the general Formula I :

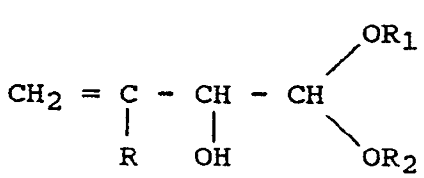

(I)

where R denotes a cyano group, a substituted or unsubstituted acyl group, a substituted or unsubstituted alkoxycarbonyl group or a substituted or unsubstituted aryloxycarbonyl group,

$R_1$ and $R_2$ are identical and each denote a —$CHR_3R_4$ group, where $R_3$ and $R_4$ are identical or different and

denote a hydrogen atom or an alkyl, alkenyl or aralkyl group, or

$R_1$ and $R_2$ together form a bivalent radical —$CH_2$-$(CR_5R_6)_n$-$CHR_7$— where n is 0 or 1 and $R_5$, $R_6$ and $R_7$ are identical or different and denote a hydrogen atom or a methyl group.

2. An ethylenic monomer as defined in claim 1, characterized in that in the Formula (I) R denotes a cyano group or an alkoxycarbonyl group and $R_1$ and $R_2$, when identical, each denotes a methyl, ethyl or butyl group, or $R_1$ and $R_2$ together form a bivalent radical such as ethylene, trimethylene, propylene or 2,2-dimethyl-1,3-propanediyl.

3. 2-cyano-4,4-dimethoxy-3-hydroxy-1-butene.

4. 2-cyano-4,4-dibutoxy-3-hydroxy-1-butene.

5. 4,4-dibutoxy-2-ethoxycarbonyl-3-hydroxy-1-butene.

6. 2-butoxycarbonyl-4,4-dibutoxy-3-hydroxy-1-butene.

7. 4,4-dimethoxy-3-hydroxy-2-hydroxyethoxycarbonyl-1-butene.

8. 4,4-dimethoxy-2-dimethylaminoethoxycarbonyl-3-hydroxy-1-butene.

9. 4,4-dimethoxy-2-ethoxycarbonyl-3-hydroxy-1-butene.

**Claims for the following Contracting State : ES**

1. Process for the preparation of a polyfunctional ethylenic monomer having the general Formula I :

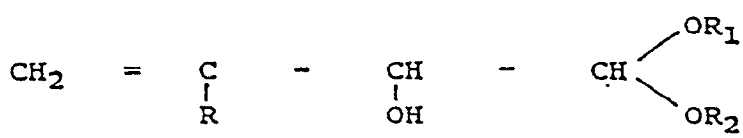

(I)

where R denotes a cyano group, a substituted or unsubstituted acyl group, a substituted or unsubstituted alkoxycarbonyl group or a substituted or unsubstituted aryloxycarbonyl group,

$R_1$ and $R_2$ are identical and each denote a —$CHR_3$ $R_4$ group, where $R_3$ and $4_4$ are identical or different and denote a hydrogen atom or an alkyl, alkenyl or aralkyl group, or

$R_1$ and $R_2$ together form a bivalent radical —$CH_2$ -$(CR_5$ $R_6)_n$ -$CHR_7$— where n is 0 or 1 and $R_5$, $R_6$ and $R_7$ are identical or different and denote a hydrogen atom or a methyl group,

characterised in that a glyoxal monoacetal of Formula (II) :

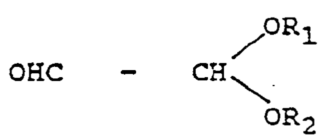

(II)

where $R_1$ and $R_2$ are as defined above is reacted with an ethylenic derivative of Formula (III) :

$$H_2C = CH - R \qquad (III)$$

where R is as defined above, in the presence of an aprotic alkaline catalyst.

2. A process as defined in claim 1, characterised in that the catalyst is [2,2,2] - diazabicyclo-1,4-octane.

3. A process as defined in claim 1 or 2, characterised in that a derivative of Formula (I) is prepared wherein R denotes a cyano group or an alkoxycarbonyl group and $R_1$ and $R_2$, when identical, each denotes a methyl, ethyl or butyl group, or $R_1$ and $R_2$ together form a bivalent radical such as ethylene, trimethylene, propylene or 2,2-dimethyl-1,3-propanediyl.

4. A process as defined in claim 1 or 2, characterised in that 2-cyano-4,4-dimethoxy-3-hydroxy-1-butene is prepared.

5. A process as defined in claim 1 or 2, characterised in that 2-cyano-4,4-dibutoxy-3-hydroxy-1-butene is prepared.

6. A process as defined in claim 1 or 2, characterised in that 4,4-dibutoxy-2-ethoxycarbonyl-3-hydroxy-1-butene is prepared.

7. A process as defined in claim 1 or 2, characterised in that 2-butoxycarbonyl-4,4-dibutoxy-3-hydroxy-1-butene is prepared.

8. A process as defined in claim 1 or 2, characterised in that 4,4-dimethoxy-3-hydroxy-2-hydroxyethoxycarbonyl-1-butene is prepared.

9. A process as defined in claim 1 or 2, characterised in that 4,4-dimethoxy-2-dimethylaminoethoxycarbonyl-3-hydroxy-1-butene is prepared.

10. A process as defined in claim 1 or 2, characterised in that 4,4-dimethoxy-2-ethoxycarbonyl-3-hydroxy-1-butene is prepared.